# EUROPEAN PATENT APPLICATION

(11) **EP 4 682 762 A1**
(43) Date of publication of application: **21.01.2026**
(21) Application number: 24382771.4
(22) Date of filing: 16.07.2024
(51) Int. Cl.: G06N 3/045, G06N 3/0475, G06N 3/094, G06N 10/60, G16C 20/00, G16C 60/00

(54) **TRAINING METHOD FOR GENERATIVE ADVERSARIAL NETWORKS FOR DATA GENERATION**

(71) Applicant: Qcentroid Labs , S.L., 48001 Bilbao (Bizkaia) (ES)
(72) Inventor: Kuchkovsky, Carlos, 48001 Bilbao (ES); Novo, Oscar, 48001 Bilbao (ES)
(74) Representative: Pons IP

(57) **Abstract**

A training method for Generative Adversarial Networks (GAN) for data generation comprising the steps of initializing a generator of the GAN; inputting semantic meta-data which comprises the definition of conditional restrictions on the internal parameters of the generator of the GAN to align with characteristics of the real data; generating output data by using the generator of the GAN; feeding a discriminator of the GAN with the output data generated or real data; determining by using the discriminator if data fed is output data generated or real data; feeding the generator with the determination of the discriminator; and training the generator and the discriminator by repeating the previous steps.

## Description

### OBJECT OF THE INVENTION

The invention is related to the field of Generative Artificial Intelligence (GAN) models, more particularly to data generation using GAN models.

The object of the invention is a system and method using a training approach for Generative Adversarial Networks (GAN) for data generation, which includes the semantic information within the GAN model to improve the production of new content more precisely and needing less dataset for the training.

### BACKGROUND ART

Generative AI encompasses several notable techniques, each with its own approach to creative data generation. Some of the most well-known techniques are Generative Adversarial Networks (GANs), Variational Autoencoders (VAEs), Recurrent Neural Networks (RNNs) and transformers:
- Generative Adversarial Networks (GANs): Introduced in 2014, GANs consist of a generator and a discriminator engaged in a competitive learning process. The generator creates data, while the discriminator assesses its authenticity. Over time, GANs produce an increasingly realistic output.
- Variational Autoencoders (VAEs): VAEs employ probabilistic modeling to learn and generate data. They are particularly effective in encoding and decoding complex data distributions.
- Recurrent Neural Networks (RNNs): RNNs are well-suited for sequential data generation tasks, making them invaluable in generating text, music, and even video captions.
- Transformer Models: Transformer models, like GPT (Generative Pre-trained Transformer), are used in natural language generation tasks. They generate readable and contextually relevant text, making them essential in applications like chatbots and language translation.

The aforementioned models have been instrumental across numerous fields, including art, healthcare, and chemistry. Notably, in domains like drug discovery, molecular design, and the creation of original artwork, these models have pushed the boundaries of human creativity to unprecedented heights.

On the other hand, Generative Adversarial Networks, commonly referred to as GANs, represent a groundbreaking approach to generative artificial intelligence. These networks have become a cornerstone of the field. GANs are particularly unique due to their structure and dynamic training process.

At its core, Generative Artificial Intelligence (Generative Al) is about providing machines with the ability to create data that closely mimics real-world information. Instead of merely analyzing or classifying existing data, generative Al strives to expand the horizon of what machines can generate. It empowers Al systems to produce content such as images, text, and/or music.

At the heart of GANs lies a dynamic interplay between two neural networks: the Generator and the Discriminator:
- Generator: The Generator's primary role is to, as the name suggests, generate synthetic data. It starts with random noise and attempts to transform it into data that closely resembles real-world information. Initially, the output of the Generator is often far from convincing or realistic but after several iterations with the Discriminator, its output improves drastically.
- Discriminator: The Discriminator's task is to distinguish between real data and the data generated by the Generator. The Discriminator's objective is to become skilled at differentiating between genuine data and the synthetic data generated by the generator.

The power of GANs unfolds during the adversarial training process, where the Generator and Discriminator continuously compete and improve. Here's how it works:
- Training Phase: Initially, both the Generator and the Discriminator are relatively unskilled. The Generator produces data that is far from authentic, and the Discriminator makes errors in distinguishing real from synthetic.
- Competitive Learning: During each training iteration, the Generator aims to create data that becomes increasingly realistic. Simultaneously, the Discriminator improves its ability to tell the real data apart from the generated data.
- Equilibrium: Over time, a balance is found. The Generator becomes adept at creating data that is difficult for the Discriminator to differentiate from real data. This equilibrium is where GANs produce high-quality synthetic data.

Nevertheless, while Generative Adversarial Networks (GANs) offer numerous advantages and find applications in various domains, they do come with several limitations.

One of the primary constraints pertains to the generative network's reliance on generating data. For instance, in many use cases of images, the generative network generates each pixel independently. Consequently, it takes more time for the network to converge from producing less realistic images to generating ones that closely resemble real-world counterparts.

Furthermore, GANs often struggle to capture specific attributes that are easily discernible to humans. For instance, human hands typically have five fingers, and human faces possess distinct aesthetic features which are difficult to generate from many GAN models. Replicating or generating such details, like the symmetry of eyes, ears, or teeth, can prove challenging for GANs.

In many cases, addressing these misleading data artifacts needs extensive training iterations between the generator and discriminator networks. Achieving effective results typically involves pre-training the network with a substantial and diverse dataset, often ranging from 70,000 to 100,000 data points. However, securing such extensive and varied datasets can pose significant challenges, especially in specialized domains like chemical reactions where there are not many specific reactions. Consequently, in these specific use cases, GANs may struggle to consistently generate accurate data representations.

Thus, there is not in the art a solution which allows to consistently generate accurate data representations with a reduced training dataset.

### DESCRIPTION OF THE INVENTION

The present invention discloses a training method for Generative Adversarial Networks (GAN) for data generation which, by defining the semantic meta-data inside the model, allows to produce more precise results enhancing the efficiency of result data generation.

The training method for Generative Adversarial Network (GAN) data generation comprises the steps of:
a) initializing a generator of the GAN;
b) inputting semantic meta-data, which constrains the output data of the generator;
c) generating output data by using the generator of the GAN;
d) feeding a discriminator of the GAN with the output data generated or real data;
e) determining by using the discriminator if data fed is output data generated or real data;
f) feeding the generator with the determination of the discriminator;
g) training the generator and the discriminator by repeating the steps c) to f);

More particularly, the step of inputting semantic meta-data comprises the definition of conditional restrictions on the internal parameters of the generator of the GAN to align with characteristics of the real data.

By defining the semantic meta-data inside the model, the generator can produce proper outcomes and the data can be achieved faster and be more realistic.

Generative Adversarial Networks (GANs) are primarily utilized in image generation tasks. However, their output may sometimes lack accuracy in, for example, proportions due to the GANs' limited understanding of real-world semantics. For instance, human hands typically feature five fingers, and there exists a specific symmetry between the distances of the eyes and ears on a human face. Hence, GANs require a comprehensive and varied dataset to grasp these features effectively. In cases where the dataset lacks diversity, the network may fail to learn these crucial features.

In a preferred set of embodiments, the real data and the output data generated could be an image represented by a matrix of pixels. In these cases, the semantic meta-data comprises proportionality relations between pixels. Said proportionality relations could be preferably proportionality relations. More precisely, the semantic meta-data could comprise face proportionality relations selected from: eye distance, eyebrow distance, hear line distance, nose-lips distance and bottom to chin distance. Also, other types of proportionality relations could be incorporated, such as landscape or building proportionality relations.

Moreover, the semantic meta-data could further comprise data regarding the number of legs, eyes, ears, noses, mouths, heads and other body parts, or numerical relationships such as the number of tires on a car or a bike, or the number of blades on a fan.

Furthermore, GANs have potential applications beyond image generation, such as in chemistry reactions.

In an alternative set of embodiments, the real data and the output data generated could be a catalysis reaction.

In this case, the real data and the output data generated could comprise:
- an absorbent, comprising a set of elements, and
- a catalysis surface comprising a set of elements.

The semantic meta-data would comprise a restriction that prevents the generator from generating synthetic elements of certain types as a result of the catalysis reaction. In this case, the restrictions would be applied on:
- one or more types of chemical elements of the absorbent and/or one or more types of chemical elements of the catalyst surface, and/or
- the number of elements on the catalyst surface.

With this semantic meta-data, the generator would generate catalysis reactions according to the restriction imposed by said semantic meta-data. It should be noted that synthetic elements produced by a generator refer to artificially created data points or instances that mimic real-world data. These chemical elements are generated by **the GAN's algorithms to resemble the characteristics and patterns of the training data.** The data generation performed by the generator transforms random data into a synthetic catalyst reaction that resembles real reactions to feed the discriminator.

In the context of the present invention, the surface and absorbent of a catalysis reaction could be represented as arrays of numbers. In these cases, the generator could further generate other physical or chemical data related to the catalysis reaction, such as energy.

In particular, the arrays could comprise:
- the absorbent array represents the chemical elements within the absorbent, with each element indicating the atomic number of each individual atom, and
- the surface array represents the chemical elements within the surface, with each element indicating the atomic number of each individual atom.

Thus, the restriction on the semantic meta-data would be on:
- **one or more chemical element's atomic numbers of the absorbent and/or one or more chemical element's atomic numbers of the catalyst surface, and/or**
- the number of elements on the catalyst surface.

In this scenario, the generator generates values for each element of the arrays, representing the atomic numbers of the chemical elements present on the surface and absorbent of the catalyst reaction
As there is no semantic data associated with this model, the generator will generate any type of data, causing a longer convergence time with the discriminator, which possesses the real data. Moreover, the database must be extensive and varied for the discriminator to effectively distinguish the correct output.

In the previous scenario, the model lacks the semantic information that humans possess to redefine faster the generated data.

Thus, the invention adds semantic meta-data to incorporate the semantic information needed to accelerate the convergence of the model. For example, in addition to the previously described semantic data in images, the semantic meta-data could comprise thermodynamic laws or define specific chemical reactions.

In a preferred set of embodiments, if the training database is related to catalysis reactions, this knowledge can be systematically integrated into the generator, resulting in quicker and more precise generated outputs.

Preferably, the semantic meta-data comprises a restriction on one or more chemical elements of the absorbent and/or one or more chemical elements of the catalyst surface. These chemical elements could be represented by its atomic numbers, and, in this case, the semantic meta-data would comprise a restriction in said atomic numbers. Thus, the generator generates data according to the restrictions of the semantic meta-data.

In some embodiments, the semantic meta-data comprises a restriction on one or more of the chemical elements of the absorbent. In such a case, the generator generates values for the chemical elements of the absorbent not restricted. Also, the generator generates values for the chemical elements of the catalyst surface and for the number of atoms of each chemical element in the catalyst surface.

In an alternative embodiment, the semantic meta-data could comprise a restriction on one or more chemical elements of the catalyst surface. In this case, the generator generates values for the chemical elements of the absorbent and for the chemical elements of the catalyst surface not restricted. Also, the generator determines values for the number of atoms of each chemical element in the catalyst surface, restricted and not restricted.

If the semantic data is limited to alloys of a specific number of elements, for example, binary, tertiary or quaternary alloys for catalysis, then the semantic meta-data includes a restriction on the total number of chemical elements on the catalyst surface. For example, if the generator is set for binary catalysis, it produces the data for the two elements of the catalyst surface, such as the atomic values.

Additionally, the generator generates data for the number of atoms of each of the chemical elements on the catalyst reactions. This process similarly applies to tertiary, quaternary, or any other number of alloys with the corresponding number of elements.

In the method of the invention, the generator could comprise a quantum layer placed in a middle layer of its architecture. Preferably, said quantum layer could comprise one or more Parametrized Quantum Circuits (PQC), each one comprising multiple quantum gates and being configured to encode classical data into quantum states, more specifically, the semantic meta-data could be encoded into quantum bits.

Additionally, the generator could also comprise a Quantum Noise Generator at its input, configured to introduce randomness and leverage quantum characteristics within input data of the generator.

In addition to that, the invention can also be applicable in a quantum system comprising a processing unit configured to perform the steps of the method described and to encode data into quantum bits and quantum registers.

The invention also relates to a computer program adapted to perform the steps of the method of the invention and a computer readable storage medium comprising said computer program.

The method of the invention facilitates applying requirements as part of the GAN model for generating images. Thus, the training of the model can be done faster and with smaller training datasets. Also, the generator and discriminator networks converge easier, and the results are more realistic and follow more closely to the specifications of the final solution.

The invention also relates to a quantum system comprising a processing unit configured to perform the steps of the method previously defined and to encode the data into quantum bits and quantum registers.

### DESCRIPTION OF THE DRAWINGS

Fig. 1. Shows an illustration of a GAN architecture, comprising a Generator and a Discriminator. The Generator, fed initially by noise, generates synthetic data, while the Discriminator, which is fed with real data and synthetic data, distinguishes between real and synthetic.
Fig. 2 Shows a collection of images illustrating asymmetry in human faces generated by GAN models, including variations in teeth alignment and unrealistic eye and ear distances.
Figure 3. Shows an example of proportionality relations that could be used as semantic meta-data for image generation according to the method of the invention.
Fig. 4. Shows an example of a Catalysis reaction of oxygen and carbon monoxide to form carbon dioxide, showing the phases of: absorption, chemical reaction, and desorption.
Figure 5. Shows an illustration of a GAN architecture, comprising the Generator and the Discriminator applied to a catalysis reaction generation. The Generator, fed initially by noise, generates a synthetic catalysis reaction, while the Discriminator, which is fed with real catalysis reaction and the generated catalysis reaction, distinguishes between real and generated.
Figure 6. Shows an example of the training method without incorporating semantic meta-data.
Figure 7. Shows an example of the training method according to the invention incorporating semantic meta-data, wherein the generation step is restricted to generate Copper-based binary alloy catalysis. The example illustrates a binary alloy composed of 11 atoms of copper, which has an atomic number of 29, and 10 atoms of fluorine, which has an atomic number of 9.

### PREFERRED EMBODIMENTS OF THE INVENTION

The invention relates to a training method for Generative Adversarial Networks (GAN) for data generation for increasing the precision of a GAN model.

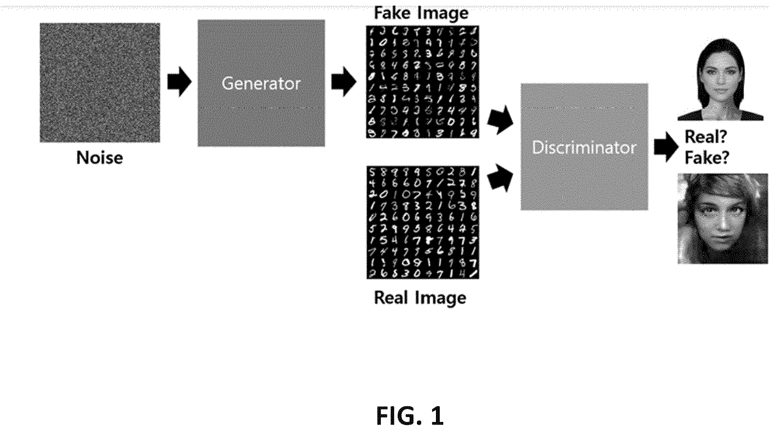

Figure 1 shows an illustration of a GAN architecture, where a Generator and a Discriminator are in a continual battle. The Generator creates synthetic or generated data, while the Discriminator distinguishes between real and generated data, resulting in a learning process that generates increasingly realistic content.

In the training method for Generative Adversarial Networks (GAN) for data generation of the invention a step of inputting semantic meta-data is incorporated such that the semantic meta-data defines conditional restrictions on the internal parameters of the generator of the GAN to align with characteristics of the real data. Thus, the main core of this invention is the generation of defining the requirements (or semantics) of the generated data inside the model. That way, the generator can produce proper outcomes and the data can be achieved faster and be more realistic.

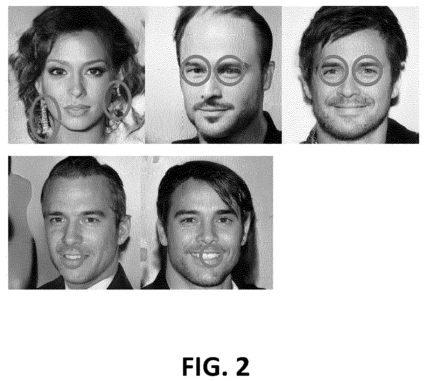

As previously discussed, it is quite common for Al-generated images to exhibit flaws and deviate from established facial proportions when the database used for training is small or lacks diversity. In such cases, errors, such as facial asymmetry or weird teeth, may occur as depicted in Figure 2.

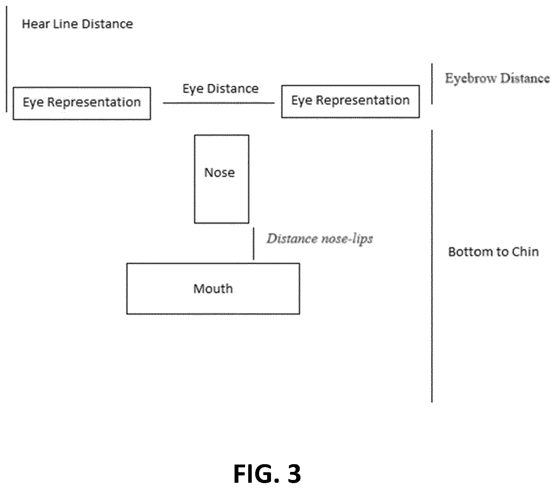

To address this issue, the approach of the invention can be employed as a solution where the facial proportions are also generated by the generator network. As depicted in Figure 3, the model can learn that proportionality and apply it to the newly generated images. Then, the discriminator network can evaluate the images to distinguish the generated images from the real ones.

The proportionality described by the generated distances (eye distance, eyebrow distance, etc.) can be used later to combine all the generated components of the image to form a perfectly proportionate face.

To provide a better understanding of how Generative Adversarial Networks (GANs) function and how the method of the invention can enhance data generation, another less conventional example is provided related to catalyst reactions generated by GAN models.

In a catalyst reaction, a catalyst is a substance that facilitates chemical reactions by creating an alternative reaction pathway with lower activation energy. This pathway allows molecules to be absorbed onto the catalyst's surface, undergo transformations, and subsequently release as new molecules. The efficiency of a catalyst is highly dependent on the specific chemical elements present in both the absorbent molecules and the catalyst's surface.

Summarizing, a catalyst reaction involves the interaction between absorbent molecules and a catalyst's surface, resulting in the release of new molecules.

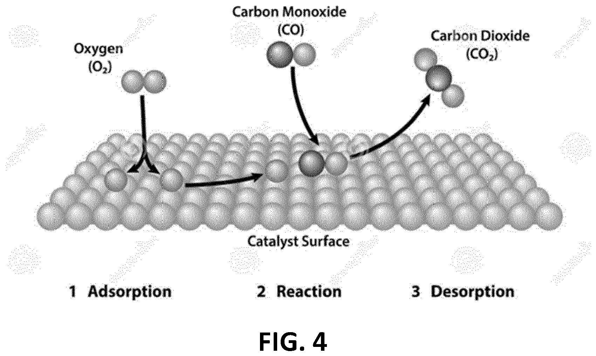

Figure 4 shows a catalyst-mediated reaction of oxygen and carbon monoxide to form carbon dioxide, depicting the sequential phases of absorption, chemical reaction, and desorption.

In the context of the invention, the representation of both the absorbent and the surface is done using arrays of a predetermined length.

The core of this approach lies in the generator network of the GAN, which generates values for these arrays. Each element within the absorbent array corresponds to a specific chemical element's atomic number, and the same principle applies to the catalyst's surface array.

For instance, consider an array with 5 elements: [5, 7, 29, 34, 100]. In this array:
- Element 5 corresponds to Boron (B).
- Element 7 corresponds to Nitrogen (N).
- Element 29 corresponds to Copper (Cu).
- Element 34 corresponds to Selenium (Se).
- Element 100 corresponds to Fermium (Fm).

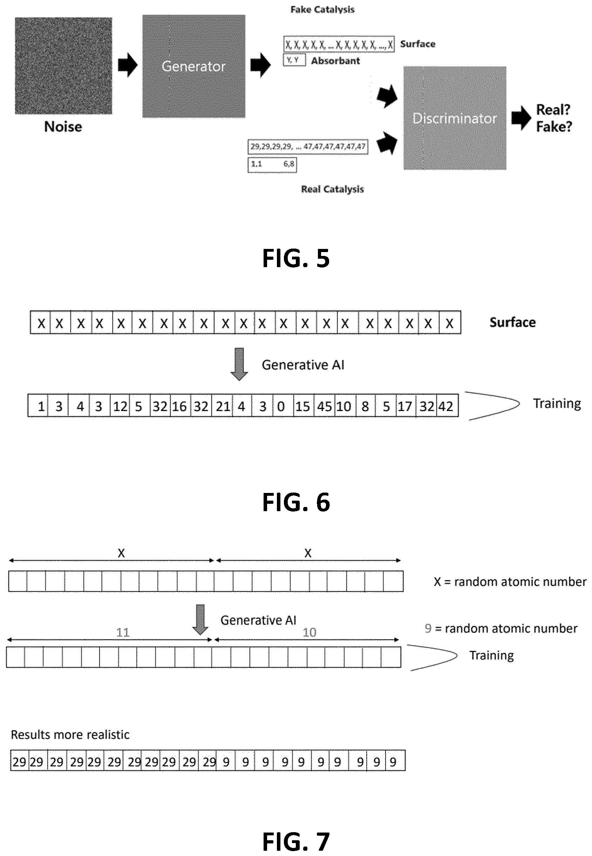

Figure 5 shows a Catalyst Exploration using GANs. In said figure it can be seen a GAN model along with the arrays generated by the generator networks, symbolizing the absorbent and surface components. Within this framework, the generator network generates values for each element in these arrays, where each element corresponds to a distinct chemical element. This example highlights how Al-driven exploration can optimize catalyst configurations for heightened catalytic efficiency.

Figure 6 illustrates the process of generating new catalysts within the generator network. In this particular instance, the focus is on the surface array, and the generator randomly generates each element of this array. After each iteration, adjustments are made to the network's internal parameters to align with the characteristics of real surface catalysts.

In a specific use case, the goal is to generate a binary alloy of copper catalyst. This means that the generated data in the catalyst surface should consist of copper and another chemical element.

However, achieving a valid result presents a unique challenge. The network had to learn that the array should only contain two elements: copper and another element, without any specific order. This complexity resulted in a prolonged convergence process for the GAN model, and a very large dataset was required for effective model training. Unfortunately, the scarcity of binary copper catalyst data in scientific literature made it nearly impossible to train the GAN with a limited database.

Therefore, the method of the invention is applied to overcome this issue. Thus, the requirements for the generated solution are translated into the generator network of the GAN model, hence, altering the architecture generation to something more semantically significant.

In this specific scenario, as depicted in Figure 7, the approach has been shifted to generating only 3 numbers (represented as X in Figure 7) rather than each individual element of the surface array. The newly generated numbers are the following:
- Number of Copper atoms in the surface array
- Generation of a new atomic element number X
- Number of atoms of the new atomic element X in the surface array

In an example, the elements generated are 11, 9 and 10, respectively. Thus, 11 represents the number of copper atoms in the array, 9 represents the new atomic element (fluorine F) which will be combined with the copper atoms to form the catalytic surface. Furthermore, the number 10 represents the number of fluorine atoms in the surface array.

By employing this approach, the generated data becomes considerably more realistic, and the GAN model achieves faster convergence, making it more efficient.

As noted in the catalysis use case, this idea can be easily extended to generate other types of data in GAN models.

Also, the method described in this document has the potential to be extended into the realm of quantum data encoding.

In particular, the quantum approach could be implemented by adapting the architecture of the generator and the discriminator used in the method of the invention. The generator and discriminator in a GAN are composed of several layers, one or several of which could be a parameterized quantum layer. Integrating these layers allows for a seamless blend of classical and quantum computations, enhancing the **network's ability to generate and discriminate more nuanced and complex patterns.**

Using this structure, the generator continually evolves to produce more realistic data, guided by the feedback from the discriminator.

The integration of quantum components facilitates the exploration of quantum phenomena to enhance the machine learning process.

In addition, a Quantum Noise Generator can be added to the architecture to introduce an element of randomness and leveraging quantum characteristics within the input data of the generator. This Quantum Noise Generator allows to produce more nuanced and diverse outputs.

## Claims

1. A training method for Generative Adversarial Networks (GAN) for data generation comprising the steps of:
a) initializing a generator of the GAN;
b) inputting semantic meta-data, which constrains the output data of the generator;
c) generating output data by using the generator of the GAN;
d) feeding a discriminator of the GAN with the output data generated or real data;
e) determining by using the discriminator if data fed is output data generated or real data;
f) feeding the generator with the determination of the discriminator;
g) training the generator and the discriminator by repeating the steps c) to f);
wherein the step of inputting semantic meta-data comprises the definition of conditional restrictions on the internal parameters of the generator of the GAN to align with characteristics of the real data.

2. The method according to claim 1, wherein the real data and the output data generated are images represented by matrix of pixels and wherein the semantic meta-data comprises proportionality relations between features in the images.

3. The method according to claim 2, wherein the semantic meta-data comprises proportionality relations for faces, landscapes, buildings.

4. The method according to claim 3, wherein the semantic meta-data comprises proportionality relations selected from: number of fingers, legs, eyes, ears, noses, mouths and heads.

5. The method according to claim 3, wherein the semantic meta-data comprises face proportionality relations selected from: eye distance, eyebrow distance, hear line distance, nose-lips distance and bottom to chin distance.

6. The method according to claim 1, wherein the real data and the output data generated are a catalysis reaction comprising:
- an absorbent, comprising a set of elements, and
- a catalysis surface comprising a set of elements,
the semantic meta-data comprising a restriction on:
• one or more types of chemical elements of the absorbent and/or one or more types of chemical elements of the catalyst surface, and/or
• the number of elements on the catalyst surface; and
wherein the generator generates catalysis reactions according to the restriction of the semantic meta-data.

7. The method according to claim 6, wherein the catalysis reaction is represented by arrays of numbers, the catalysis reaction comprising:
- an absorbent array, comprising a set of elements, each one represented by an atomic number of each individual atom in the absorbent,
- a catalysis surface array comprising a set of elements represented by an atomic number of each individual atom in the catalyst surface,
and wherein the restriction on the semantic meta-data are:
• one or more chemical element's atomic numbers of the absorbent and/or one or more chemical **element's atomic numbers** of the catalyst surface, and/or
• the number of elements on the catalyst surface; and
wherein the generator generates values for the numbers of the arrays according to the restriction of the semantic meta-data.

8. The method according to claim 6, wherein the semantic meta-data comprises a restriction on one or more of the chemical elements of the absorbent, and wherein the generator generates values for the chemical elements of the absorbent not restricted and the chemical elements of the catalyst surface and for the number of atoms of each chemical element in the catalyst surface.

9. The method according to claim 8, wherein the semantic meta-data comprises a restriction on one or more chemical elements of the catalyst surface, and wherein the generator generates values for the chemical elements of the absorbent and the chemical elements of the catalyst surface not restricted and for the number of atoms of each chemical element in the catalyst surface.

10. The method according to claim 8, wherein the semantic meta-data comprise a limitation to a specific number of elements on the catalyst surface according to a specification of using alloys of said specific number of elements.

11. The method according to claim 1, wherein the data is encoded into quantum bits.

12. The method according to claim 1, wherein the generator comprises a quantum layer placed in a middle layer of its architecture.

13. The method according to claim 12, wherein the quantum layer comprises one or more Parametrized Quantum Circuits (PQC), each one comprising multiple quantum gates and being configured to encode classical data into quantum states.

14. The method according to claim 1, wherein the generator comprises a Quantum Noise Generator at its input, configured to introduce randomness and leverage quantum characteristics within input data of the generator.

15. A quantum system comprising a quantum processing unit configured to perform the steps of the method according to any of claims 1 to 14 and to encode semantic meta-data into quantum bits and quantum registers.

## Amended claims

### Amended claims in accordance with Rule 137(2) EPC.

1. A training method for Generative Adversarial Networks (GAN) for data generation comprising the steps of:
a) initializing a generator of the GAN;
b) inputting user-defined semantic constraints, which shapes the output data of the generator;
c) generating output data in the form of semantic meta-data representation adapted to a particular use case by using the generator of the GAN;
d) feeding a discriminator of the GAN with the output data generated or real data;
e) determining by using the discriminator if data fed is output data generated or real data;
f) feeding the generator with the determination of the discriminator;
g) training the generator and the discriminator by repeating the steps c) to f); wherein the step of inputting used-defined semantic constraints comprise the definition of conditional restrictions on the internal parameters of the generator of the GAN to align with characteristics of the real data.

2. The method according to claim 1, wherein the real data and the output data generated are images represented by matrix of pixels and wherein the semantic meta-data representation comprises proportionality relations between features in the images.

3. The method according to claim 2, wherein the semantic meta-data representation comprises proportionality relations for faces, landscapes, buildings.

4. The method according to claim 3, wherein the semantic meta-data representation comprises proportionality relations selected from: number of fingers, legs, eyes, ears, noses, mouths and heads.

5. The method according to claim 3, wherein the semantic meta-data representation comprises face proportionality relations selected from: eye distance, eyebrow distance, hear line distance, nose-lips distance and bottom to chin distance.

6. The method according to claim 1, wherein the semantic meta-data representation comprises outputs related to molecule generation, such as in the design of catalysts or audio synthesis.

7. The method according to claim 1, wherein the semantic meta-data represented by the generator comprises catalytic surfaces, and chemical elements, and is represented by non-image data in use cases other than image generation.

8. The method according to claim 1, wherein the data is encoded to quantum bits.
